# EUROPEAN PATENT APPLICATION

(11) **EP 2 014 619 A2**
(43) Date of publication of application: **14.01.2009**
(21) Application number: 07109134.2
(22) Date of filing: 29.05.2007
(51) Int. Cl.: C01B 39/02, B01J 29/18, C07C 2/66

(54) **Mesoporous mordenite, preparation and use thereof**

(71) Applicant: Technische Universiteit Delft, 2628 BL Delft (NL); Institute of Chemical Research of Catalonia (ICIQ), 43007 Tarragona (ES)
(72) Inventor: Groen, Jozef Cornelis, 3135 ZC Vlaardingen (NL); Moulijn, Jacob Adriaan, 2517 HB Den Haag (NL); Pérez Ramírez, Javier, 43007 Tarragona (ES)
(74) Representative: van Loon, C.J.J.

(57) **Abstract**

The invention is directed to a process for the preparation of a mesoporous mordenite, which process comprises subjecting a non-dealuminated mordenite having an atomic ratio of framework Si-to-Al of least 15, to an alkaline treatment in order to create mesoporosity by removal of silicon, to various mesoporous mordenites and to their use.

## Description

The invention is directed to mordenites having a high mesoporosity, to the preparation of such mordenites, and to their use.

Zeolites and more in particular mordenites, are used in many catalytic and separation processes due to unique properties such as uniform pores of molecular dimensions, intrinsic acidity, and the ability to confine active metal species. However, the microporous character of these materials often leads to intracrystalline diffusion limitations, which adversely affect catalytic performance. A breakthrough was the synthesis of mesostructured aluminosilicates. Emerging mesoporous materials however generally do not comply with most practical requirements as a result of limited thermal stability and poor acidic properties.

Consequently, new synthesis procedures for preparation of small zeolitic crystals or post-treatment procedures to create extra-porosity are increasingly investigated. Conventional steaming and acid leaching methods or the less-known treatments in alkaline media have been applied to alter various properties of zeolites. The last method removes preferentially Si from the zeolite framework (desilication), while the former ones lead to dealumination. Desilication was firstly applied to study chemical changes of MFI crystals upon contact with NaOH solutions, but only recently this treatment has shown to induce a significant mesoporosity in MFI-type zeolites.

Groen et al, On the introduction of intracrystalline mesoporosity in zeolites upon desilication in alkaline medium, Microporous and Mesoporous Materials, 69 (2004) pp 29-34, describe the influence of the extraction of silicon from zeolite frameworks on their structure.

In general, results from this type of treatments often cannot be extrapolated directly from one type of zeolites to other zeolite types, or even within one family due to e.g. different framework properties, stability, and typical Si- to-A1 ratios.

Mordenites are a separate class of zeolites and are applied frequently as catalyst or catalyst support in chemical reactions, such a the alkylation of aromatic compounds, (hydro-)isomerization, esterification, dehydration of alcohols, amine production from NH₃ and olefins, acetal or ketal formation, aldol condensation reactions, and the like.

More in particular mordenite can be used as a solid acid catalyst, or as a catalyst support for those reactions wherein both acidic properties and the properties of the applied catalytically active material (bifunctional catalyst) are required. In addition thereto mordenite is also useful as a catalyst support in case the trivalent metal (aluminum) is needed as anchoring site for the catalytically active material (usually a metal).

The mordenite structure is defined in the Atlas of zeolite framework types, 5^{th} revised edition (2001). In this context the term mordenite not only includes the materials based on silicon and aluminum, but also those materials with the same structure, wherein the trivalent aluminum has been replaced in whole or in part by other trivalent atoms, such as iron, boron or gallium.

As indicated above, it would be advantageous if the pore structure of the mordenite could be improved, especially with respect to surface area in mesopores. Because of the pore structure of mordenite, consisting of relatively wide unidirectional pores and relatively narrow pores connecting these wide pores, the material is rather susceptible to disturbances of the pore structure. Increase of mesoporosity in mordenites would result in a better accessibility of the pores and would allow for transport between parallel pores, thereby making the material less susceptible to disturbances of the pore structure.

Further it would be advantageous if the catalytic properties of the mordenite in general could be improved, more in particular with respect to activity, selectivity and stability.

In the modification of mordenites it is important that the process proceeds smoothly, and that the resulting material has predictable properties in terms of structure, reproducibility of acidic character, strength and framework Si-to-trivalent metal atomic ratio in the end product. When used as catalyst or catalyst support, it is often important that the original acidity of the mordenite is maintained.

Accordingly it is an object of the present invention to provide a process for increasing the mesoporosity of mordenites (MOR), while substantially maintaining the acidity thereof, and to improve the catalytic properties thereof. The present invention is based on the surprising discovery, that in order to meet these objects it is essential that the atomic Si-to-Al ratio in the mordenite exceeds 15, preferably exceeds 20 and more preferably is between 25 and 35.

For ease of definition, whenever mention is made of aluminum herein, it is to be understood that the trivalent aluminum may have been replaced in whole or in part by other trivalent atoms, such as iron, boron or gallium, unless it is clearly indicated that only aluminum is intended.

In a first embodiment the present invention is directed to a process for the preparation of a mesoporous mordenite, which process comprises subjecting a non-dealuminated mordenite having an atomic ratio of Si-to-Al of least 15 to an alkaline treatment in order to create mesoporosity by removal of silicon. The atomic Si-to-Al ratio is defined as the ratio of the atoms in the mordenite framework. Atoms present in free alumina or silica are not included herein.

Surprisingly it has been found that in case an untreated mordenite is used with an atomic Si-to-Al ratio as defined above, the resulting mesoporous mordenite meets the requirements in terms of reproducibility and improvement of catalytic properties.

It is to be noted that the article of Groen, referred to above, mentions the alkaline treatment of a mordenite having a high Si-to-Al ratio. However, this material has previously been dealuminated. Further, the data in the article show that the effect of the treatment is relatively limited, whereas the reproducibility/predictability of the treatment is low. The conditions for the treatment described therein are further severe.

In a further embodiment the invention resides in the mesoporous mordenite produced in accordance with the above process and in a mesoporous mordenite having a surface area in mesopores of between 50 and 200 m²/g, and an acidity, as defined herein, of between 0.35 and 0.95 mmol/g.

With respect of the surface area in mesopores, it is to be noted that the measurement of this values includes some surface area on the outside of the crystals, as intercrystalline pore-volume is also included in the measurement. Accordingly, the particle size of the crystals influences the value. Generally, this outer surface area is rather limited and almost negligible. One solution would be to distract the outer surface area as determined by SEM measurements from this value. Another solution would be to define the added surface area in mesopores. This added surface area should preferably be between 45 and 150 m²/g.

The invention is also embodied in the use of the mordenite of the invention as catalyst or catalyst support for catalytic chemical reactions, such as those enumerated in the introductory part. When used as support, it has been found that the new pore structure leads to advantageous properties in terms of stabilizing the catalytically active material in the pores. Thanks to the improved pore structure, metal species (which are the most commonly used catalytically active materials) are better confined, while at the same time remaining accessible to the reactants.

Examples of catalytically active materials to be used in the present invention are the precious metals, such as Pt, Pd, Ir, Au, Rh and Ag. Other metals to be used include Ni, Co, Mn, V, Cu, Fe and Zn, to name but a few.

Important classes of reactions to be contemplated in the context of the present invention are hydro-isomerisation and the alkylation of aromatic compounds, such as the production of ethylbenzene from benzene and ethylene.

Further important advantages of the modified mordenites of the present invention are, in addition to the aspects discussed above, that by the alkaline treatment and the resulting removal of trivalent metal from the mordenite framework, various important properties of the mordenite may be modified or fine-tuned, while maintaining other, equally important, properties of the material. More in particular it is possible to modify the anchoring sites for catalytically active metals, such as the noble and transition metals, on the internal surface of the pores. The trivalent metals can function as anchoring sites and by changing the amount thereof in relation to the amount of Si, the anchoring properties can be fine tuned.

Another aspect thereof is the fine tuning of the balance between hydrophilicity and hydrophobicity, which is especially important in case of reactions in a relatively hydrophilic environment.

The starting material for the process is mordenite that has a high framework Si-to-Al (trivalent metal) ratio as prepared (i.e. not de-aluminated as in the case of Groen). These materials can be prepared as described in the literature, by direct hydrothermal synthesis, with or without the use of seed crystals, in the presence of a suitable template, such as tetraethyl ammoniumhydroxide.

The alkaline treatment of the high silica mordenite is typically performed in an aqueous environment with a pH above about 11, more preferred in 0.01 to 1.0 M alkali metal hydroxide, preferably NaOH, more preferably at about 0.2 M NaOH at 310 to 400 K, preferably at 325 to 345 K for 10 to 45 min. The treatment can be done batch wise or continuous. The temperature and time can be chosen in dependence of the required treatment level.

In a typical embodiment about 330 mg of sample were vigorously stirred in 10 ml of 0.2 MNaOH solution in a polypropylene flask for 30 min at a specific temperature. Subsequently, the reaction was quenched by submersion of the flask in an ice-water mixture, followed by filtration and thorough washing with demineralized water. After filtration and washing, the zeolites were dried (preferably at about 373 K and calcined. Calcination may be done in static air at a temperature preferably between 673 and 1000 K, such as at about 823 K. The alkaline-treated samples were converted into the H-form by three successive exchanges with an NH₄NO₃ solution. Final calcination of the mordenite may be done under the same conditions as above.

The alkali concentration may be varied between the limits given above. A higher throughput of solid product is obtained at higher concentrations. Important in this respect is to keep the ratio alkali:mordenite constant in order to have good control over the silicon extraction process. Altering the ratio will impact on the degree of silicon extraction, and similar to temperature and time, enable additional tuning of the mesoporosity. Besides NaOH, other alkali metal hydroxides or carbonates can successfully be applied, although having a slightly lower efficiency than NaOH.

The mordenite of the present invention may be characterized by its mesopore surface area, its acidity and, of course, the atomic Si-to-Al ratio. Suitable characterization methods have been defined herein below. A suitable mordenite, prepared using the process of the invention is characterized by a combination of acidity and surface area in mesopores, namely by having a surface area in mesopores of between 50 and 200 m²/g (taking into account the discussion above on this subject), and an acidity, as defined herein of between 0.35 and 0.95 mmol/g. Such a mordenite has a total pore volume of between 0.10 and 0.50 ml/g and an Si-to-Al atomic ratio of 5 to 30.

The mordenites of the present invention can suitably be used as catalysts or catalyst supports. It is to be noted that although they have been treated to remove silicon, their acidity still corresponds to the original acidity, contrary to expectations. Due to their improved surface area in mesopores, i.e. pores of between 2 and 50 nm, as defined herein below, they show improved diffusion characteristics. Also the selectivity, yield and stability (absence of decrease in activity) of these materials is excellent. As the process of the present invention allows a good reproducibility, the characteristics of the mordenites produced thereby is excellent, making them extremely suitably for use in various catalytic processes, which processes require a very well tuned catalytic activity, that should remain constant over a long period of time. Further, variations from batch to batch of catalyst are low, due to the good reproducibility of the process.

Because of their acidic properties, the mordenites of the present invention are very suitable as catalyst for acid catalyzed reactions, among which alkylation of aromatic compounds and hydro-isomerisation are important classes. Especially important is the preparation of ethylbenzene from ethylene and benzene in the presence of these mordenites. The material is also suitable as support for bifunctional catalyst, and further as general catalyst support in case the acidic sites have been covered by metal or in case the acidity plays substantially no role in the catalytic activity. The material may also be used as an additive in other catalytic processes, for example as an additive in an FCC catalyst, in order to improve olefin production.

The reaction conditions for the various catalytic reactions are well known in the art and they may be applied here too.

The invention is now elucidated on the basis of the following preparation and use examples, which are intended as exemplification only.

### EXAMPLES

### Preparation example of high silica mordenites

High-silica mordenite samples were prepared by direct hydrothermal synthesis with and without seed crystals.The seed crystals were prepared by mixing NH₄NO₃ (Wako Pure Chemical, 99%) with an aqueous solution of Al(NO₃)₃ ·9H₂O (Wako Pure Chemical, 98%) and NaOH (Merck Schuchardt, 99%). Subsequently, precipitated hydrated silica (Nipsil, Nippon Silica Ind., 88% SiO₂) and tetraethylammonium hydroxide (Aldrich, 35 wt.%) were added to the mixture and homogenized in a mortar. The chemical composition of the starting synthesis gel was as follows: Si/Al = 15, NaOH/Al = 3, TEAOH/SiO₂ = 0.23. The obtained gel was then transferred to a teflon-lined stainless steel autoclave and kept at 443 K for 3 days under static conditions. The solid product obtained was filtered, washed with deionized water, and dried at 393 K. The resulting mordenite crystals were then added (4 wt.% based on total amount of SiO₂ in synthesis gel) to the synthesis mixture of high-silica mordenite with molar composition: Si/Al = 30, NH₄NO₃/SiO₂ = 0.096, and NaOH/Al = 7. After crystallization at 343 K for 3 days the final high-silica mordenite sample was obtained (sample code MOR30).

Sample MOR20 was synthesized following a similar protocol as MOR30, but without seeding and with the following molar composition of the synthesis gel: NH₄NO₃/SiO₂ = 0.046, Si/Al = 20, and NaOH/Al = 4. The resulting solids were filtered, washed with demineralized water, dried at 393 K, and finally calcined in static air at 823 K (heating rate 3 K min⁻¹) for 5 h.

Prior to further investigations, all samples were brought into the ammonium form by three successive ion exchanges in 0.1 M NH4NO₃, followed by calcination in static air at 823 K for 5 h.

### Alkaline treatment

Alkaline treatment of the mordenite was performed in 0.2 M NaOH at 338 K for 30 min. To this end, 330 mg of sample were vigorously stirred in 10 ml of NaOH solution in a polypropylene flask for 30 min at a specific temperature. Subsequently, the reaction was quenched by submersion of the flask in an ice-water mixture, followed by filtration and thorough washing with demineralized water. After filtration and washing, the mordenites were dried at 373 K and calcined in static air at 823 K. The alkaline-treated samples were converted into the H-form by three successive exchanges in 0.1 M NH₄NO₃ solution. Final calcination of the mordenites was done in static air at 823 K during 5 h.

### Characterization

N₂ adsorption at 77 K was carried out in a Quantachrome Autosorb-6B apparatus. Samples were previously evacuated at 623 K for 16 h. The BET surface area was derived from the adsorption isotherm in the adapted relative pressure range of *p*/*p₀* = 0.01-0.1 [S. Brunauer, P.H. Emmett, E. Teller, J. Am. Chem. Soc. 60 (1938) 309.]. The micropore volume (*V*_{micro}) and the macro- and mesopore surface area (*S*ₘₑₛₒ) were determined with the *t*-plot method according to Lippens and de Boer [B.C. Lippens, J.H. de Boer, J. Catal. 4 (1965) 319.]. The mesopore size distribution was calculated from the adsorption branch of the isotherm using the Barret-Joyner-Hallenda [E.P. Barret, L.G. Joyner, P.H. Hallenda, J. Am. Chem. Soc. 73 (1951) 373] pore size model.

High-resolution low-pressure argon adsorption measurements were performed after in-situ vacuum pretreatment at 623 K for 16 h on a Micromeritics ASAP 2010 to assess the microporous properties. The Saito-Foley [A. Saito, H.C. Foley, Microporous Mater. 3 (1995) 531] pore size model was used to calculate the micropore size distribution.

Temperature programmed desorption of ammonia (NH₃-TPD) was carried out in a Micromeritics TPR/TPD 2900 equipped with a thermal conductivity detector (TCD). The sample (30 mg) was pretreated at 873 K in He for 1 h. Afterwards, pure NH₃ (25 cm³ min⁻¹) was adsorbed at 473 K for 10 min. Subsequently, a flow of He (25 cm³ min⁻¹) was passed through the reactor during 25 minutes to remove weakly adsorbed NH₃ from the zeolite. This procedure was repeated three times. Desorption of NH₃ was monitored in the temperature range of 473-873 K with a ramp rate of 10 K min⁻¹ (defined as mmol NH₃ adsorbed per gram of zeolite.

The chemical composition and textural characteristics of the various zeolites are summarized in Table 1.

**Table 1. Physicochemical properties of the parent and alkaline-treated zeolites.**

| Sample | Si/Al^{a} mol mol⁻¹ | *S*_{BET}^{b} m² g⁻¹ | *S*ₘₑₛₒ ^{c} m² g⁻¹ | *V*_{micro}^{c} cm³ g⁻¹ | *V*ₜₒₜₐₗ^{d} cm³ g⁻¹ | NH₃-uptake mmol g⁻¹ |
|---|---|---|---|---|---|---|
| MOR10* | 10 | 535 | 35 5 | 0.20 | 0.31 | 1.18 |
| MOR20 | 21 | 500 | 8 | 0.21 | 0.22 | 0.80 |
| MOR30 | 27 | 520 | 5 | 0.21 | 0.22 | 0.48 |
| MOR10-*at* | 10 | 465 | 45 | 0.17 | 0.32 | 0.98 |
| MOR20-*at* | 17 | 570 | 90 | 0.20 | 0.35 | 0.78 |
| MOR30-*at* | 19 | 570 | 115 | 0.19 | 0.38 | 0.51 |

| | | | | | | |
|---|---|---|---|---|---|---|
| ^{a}ICP-OES; b BET method; ^{c} t-plot method; ^{d} Determined at *p*/*p*₀ = 0.99. * MOR10 is a commercial zeolite obtained from Zeolyst with original sample code "CBV20A". | | | | | | |

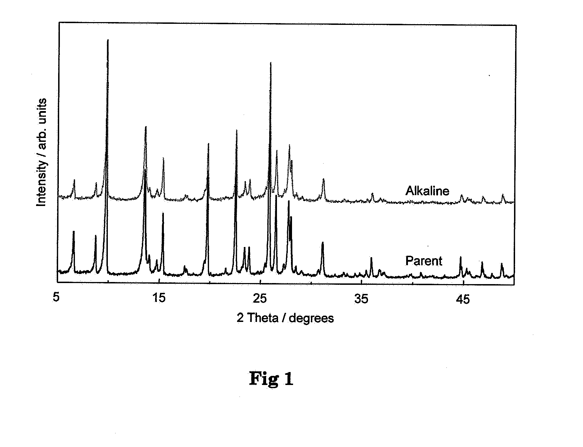

Important features of the alkaline-treated high-silica mordenite samples are the high degree of mesoporosity coupled to a mostly preserved micropore volume and acidity. XRD patterns in Figure 1 confirm the high crystallinity of the synthesized mordenite sample, with sharp reflections at the characteristic 2-theta positions. The alkaline-treated sample still presents the characteristic fingerprint, though having a slightly lower intensity, obviously as a result of the introduced mesoporosity.

### Alkylation of benzene

The activity of the various zeolites was tested in the liquid-phase alkylation of benzene (Aldrich, >99%) with ethylene (Hoekloos, >99.9%) in a 500 cm³ commercial titanium batch autoclave (Premex). First, 200 cm³ of benzene was introduced in the reactor with approximately 1 g of catalyst that was previously pretreated at 573 K in He for 12 h to remove moisture. After purging with N₂, the reactor was heated to 438 K under vigorous mechanical stirring (1000 rpm). Subsequently ethylene was introduced in the reactor until the molar ratio benzene:ethylene was 4:1 and a pressure of 23 bar was obtained. During the reaction, liquid sample aliquots (0.2 cm³) were extracted from the reactor, which were analyzed off-line with a GC (Chrompack CP9001) equipped with a CPSil-8B column and an FID detector.

Table 2 shows the differences in coke content and its impact on the textural properties of the various catalysts.

**Table 2. Textural properties of the MOR30 zeolites before and after alkylation.**

| Sample | *S*_{BET}^{a} m²g⁻¹ | *S*ₘₑₛₒ^{b} m²g⁻¹ | *V*_{micro} ^{b} cm³g⁻¹ | *V*ₜₒₜₐₗ^{c} cm³g⁻¹ | Coke wt.% |
|---|---|---|---|---|---|
| MOR30 | 500 | 5 | 0.21 | 0.22 | - |
| MOR30-used | 73 | 10 | 0.03 | 0.07 | 9.5 |
| MOR30-*at* | 570 | 115 | 0.19 | 0.38 | - |
| MOR30-*at*-used | 344 | i40 | 0.08 | 0.37 | 4.5 |

| | | | | | |
|---|---|---|---|---|---|
| ^{a}BET method; ^{b}*t*-plot method; ^{c}Determined at *p*/*p*₀ = 0.99; ^{d}Determined by thermogravimetric analysis. | | | | | |

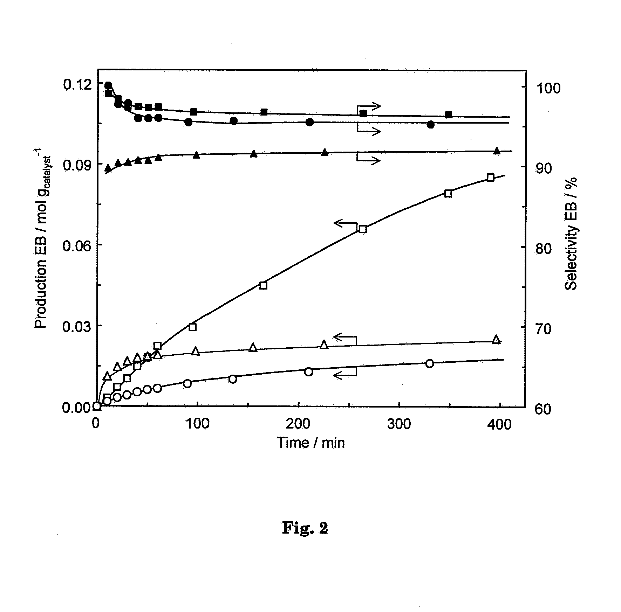

In figure 2, the results have been given of the alkylation of benzene.

In the figure the following has been shown:
ethylbenzene (EB) production (open symbols) and selectivity (filled symbols) of the different mordenite catalysts.
(o,•) MOR10, (□,▲) MOR30, and (□,■) MOR30-at.

The reported selectivity is based in the selectivity towards ethylbenzene compared to the total production of ethylbenzene, diethylbenzene, triethylbenzene, and butylbenzene together.

## Claims

1. Process for the preparation of a mesoporous mordenite, which process comprises subjecting a non-dealuminated mordenite having an atomic ratio of framework Si to Al of least 15, to an alkaline treatment in order to create mesoporosity by removal of silicon.

2. Process according to claim 1, wherein the atomic Si to Al ratio is at least 20, more preferably between 25 and 35

3. Process according to claim 1 or 2, wherein the final mordenite has an atomic Si to Al ratio lower than the original mordenite but at most 30.

4. Process according to claim 1-3, wherein the surface area of mesopores in the treated mordenite is at least 50 m²/g.

5. Process according to claim 1-4, wherein the alkaline treatment is done using an aqueous alkaline solution, preferably having a pH of at least 11.

6. Process according to claim 5, wherein the said aqueous alkaline solution is an aqueous NaOH solution of at least 0.01 M.

7. Mesoporous mordenite produced by the process of claims 1-6.

8. Mesoporous mordenite, having a surface area in mesopores of between 50 and 200 m²/g, and an acidity, as defined herein, of between 0.35 and 0.95 mmol/g.

9. Use of a mesoprous mordenite according to claim 7 or 8 as catalyst or catalyst support in a catalyzed chemical reaction.

10. Use according to claim 9, wherein the said catalytic chemical reaction is the alkylation of aromatic compounds, (hydro-)isomerization, esterification, dehydration of alcohols, amine production from NH₃ and olefins, acetal or ketal formation, or an (aldol-type) condensation reaction.

11. Process for catalytically alkylating an aromatic ring, which process comprises using a mesoporous mordenite according to claims 7 or 8 as the catalyst.

12. Process for preparing ethylbenzene, which process comprises reacting ethylene and benzene in the presence of a mesoporous mordenite according to claim 7 or 8.
